(19) Europäisches Patentamt
European Patent Office
Office européen des brevets

(11) Veröffentlichungsnummer: **0 355 006 B1**

(12) **EUROPÄISCHE PATENTSCHRIFT**

(45) Veröffentlichungstag der Patentschrift: **21.04.93**

(51) Int. Cl.[5]: **G03G 9/097**, C07D 487/04, C07D 209/44, C07D 471/04

(21) Anmeldenummer: **89114513.8**

(22) Anmeldetag: **05.08.89**

(54) **Elektrophotographischer Toner.**

(30) Priorität: **19.08.88 DE 3828193**

(43) Veröffentlichungstag der Anmeldung:
**21.02.90 Patentblatt 90/08**

(45) Bekanntmachung des Hinweises auf die Patenterteilung:
**21.04.93 Patentblatt 93/16**

(84) Benannte Vertragsstaaten:
**CH DE GB IT LI**

(56) Entgegenhaltungen:
**EP-A- 0 154 740**
**EP-A- 0 233 544**

**PATENT ABSTRACTS OF JAPAN, Band 10, Nr. 188 (P-473)[2244], 3 Juli 1986;& JP-A-61 34 554**

**JOURNAL OF HETEROCYCLIC CHEMISTRY, Band 15, Nr. 1, Januar 1978, Seiten 33-37, Hetero Corp., Provo, Utah, US; L.R. CASWELL et al.: "Cyclic imides. XL. (1) Bisimide charge-transfer polymers"**

(73) Patentinhaber: **BAYER AG**

**W-5090 Leverkusen 1 Bayerwerk(DE)**

(72) Erfinder: **Harnisch, Horst, Dr.**
**Heinenbusch 4**
**W-5203 Much(DE)**
Erfinder: **Raue, Roderich, Dr.**
**Berta-von-Suttner-Strasse 48**
**W-5090 Leverkusen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 355 006 B1

## Beschreibung

Die Erfindung betrifft positiv geladene elektrophotographische Toner, die neben üblichen Harz- und Pigmentpartikeln ein die kationische Ladung verstärkendes Additiv der allgemeinen Formel

$(X^1)^- \overset{+}{K}{}^1\text{-}Y^1\text{-}A\text{-}Y^2\text{-}\overset{+}{K}{}^2\ (X^2)^-$ (I)

enthalten, worin

| | |
|---|---|
| $(X^1)^-$ und $(X^2)^-$ | unabhängig voneinander für ein Anion, |
| $\overset{+}{K}{}^1$ und $\overset{+}{K}{}^2$ | unabhängig voneinander für |

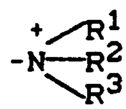 ,

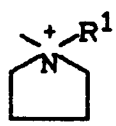 , 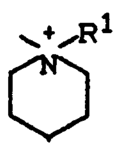 , 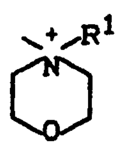 ,

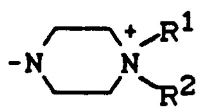 oder 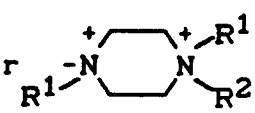 $(X^1)^-$ ,

| | |
|---|---|
| $R^1$ | für $C_1$-$C_{22}$-Alkyl, Benzyl, Phenyl, Cyclohexyl oder Allyl, |
| $R^2$ | für Wasserstoff oder $C_1$-$C_4$-Alkyl, |
| $R^3$ | für $C_1$-$C_4$-Alkyl und |
| $Y^1$ und $Y^2$ | unabhängig voneinander für $C_2$-$C_5$-Alkylen oder $_*C_6H_4$-$CH_2$- (-m oder -p) stehen, wobei die mit * gekennzeichnete Bindung mit A verbunden ist, und |
| A | ein Tetracarbonsäurediimid der Formeln |

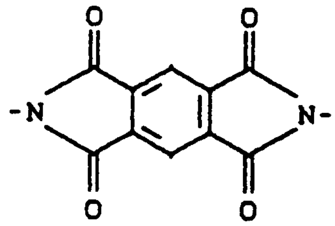 , 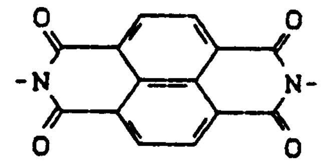 ,

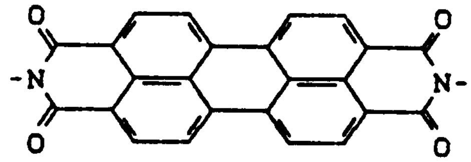 oder

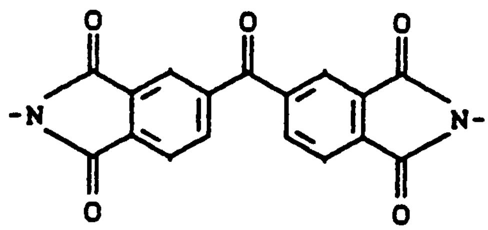

oder ein 3-Amino-1-imino-isoindolenin der Formel

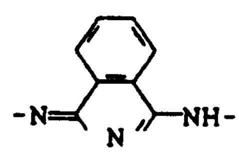

bedeutet,

und worin die cyclischen und acyclischen Reste übliche nichtionische Substituenten tragen können,

die Verwendung der Verbindungen (I) in elektrophotographischen Toner und neue Verbindungen der allgemeinen Formel

$(X^1)^- \overset{+}{K}{}^3\text{-}Y^3\text{-}A\text{-}Y^4\text{-}\overset{+}{K}{}^4\ (X^2)^-$ (II),

worin

$\overset{+}{K}{}^3$ und $\overset{+}{K}{}^4$ unabhängig voneinander für

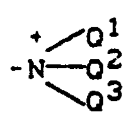

oder

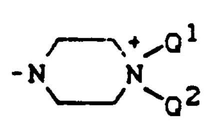 ,

| | |
|---|---|
| $Q^1$ | für $C_8$-$C_{18}$-Alkyl, |
| $Q^2$ | für Wasserstoff, Methyl oder Ethyl, |
| $Y^3$ und $Y^4$ | unabhängig voneinander für $C_2$-$C_5$-Alkylen stehen, und |
| $(X^1)^-$, $(X^2)^-$ und A | die gleiche Bedeutung wie in Formel (I) besitzen. |

Besonders geeignete Toner enthalten symmetrische Verbindungen der Formel (I), worin

$(X^1)^- = (X^2)^-$, $\overset{+}{K}^1 = \overset{+}{K}^2$ und $Y^1 = Y^2$ sind.

Von besonderem technischen Wert sind Toner, die symmetrische Verbindungen der Formel (II) enthalten, worin

$(X^1)^- = (X^2)^-$, $\overset{+}{K}^3 = \overset{+}{K}^4$ und $Y^3 = Y^4$ sind.

Geeignete nichtionische Substituenten an cyclischen und acyclischen Resten sind beispielsweise $C_1$-$C_4$-Alkyl, Halogen wie Chlor und Brom, Hydroxy, $C_1$-$C_4$-Alkoxy, $C_1$-$C_4$-Alkoxycarbonyl-, Carbamoyl- oder Sulfamoylreste, die durch 1 bis 2 $C_1$-$C_4$-Alkylreste substituiert sein können, oder Phenylreste.

Besonders geeignete Substituenten an Alkylresten sind Chlor, Hydroxy, $C_1$-$C_4$-Alkoxy, $C_1$-$C_4$-Alkoxycarbonyl- und Carbamoylreste.

Bevorzugte Reste $R^1$ sind $C_1$-$C_{22}$-Alkyl, Carbamoyl-$C_1$-$C_2$-alkyl, $C_1$-$C_4$-Alkoxycarbonyl-$C_1$-$C_2$-alkyl, Benzyl, Cyclohexyl und Allyl.

$R^2$ und $R^3$ stehen bevorzugt für $C_1$-$C_4$-Alkylreste.

Als Anionen sind übliche Anionen wie Halogenide, z.B. Chlorid, Bromid und Iodid, Tetrafluoroborate und Anionen von Alkyl- und Aryl-sulfonsäuren, -carbonsäuren, -phosphorsäuren und -phosphonsäuren geeignet. Besonders geeignet sind solche Anionen, die die Wasserlöslichkeit der Verbindungen (I) erniedrigen. Die Verminderung der Wasserlöslichkeit kann aber auch dadurch erfolgen, daß man den Alkylrest $R^1$ vergrößert, also etwa im Bereich von $C_8$-$C_{22}$-Alkyl wählt. In diesem Falle sind auch hydrophilere Anionen wie Halogenide bestens geeignet.

Die bevorzugte Wasserlöslichkeit der Verbindungen (I) bei 20 °C liegt bei unter 3 Gew.-%, insbesondere unter 1 Gew.-%. Bevorzugte Anioen sind außer Halogeniden und Tetrafluoroboraten insbesondere Arylsulfonate, wie gegebenenfalls durch $C_1$-$C_{12}$-Alkyl oder Chlor substituierte Benzolsulfonate, $C_5$-$C_{18}$-Alkylsulfonate, Salze von $C_5$-$C_{18}$-Alkylcarbonsäuren und von Kondensationsprodukten aus Formaldehyd und Arylsulfonen und/oder gegebenenfalls sulfoniertem 4,4′-Dihydroxydiphenylsulfon sowie Anionen von Heteropolysäuren auf Basis Wolfram und/oder Molybdän mit Phosphor oder Silicium, insbesondere Phosphorwolframmolybdate.

Die Verbindungen der Formel (I) können nach an sich bekannten Methoden, z.B. nach US-PS 3 544 303, DE-AS 35 35 496, Chem. Abstr. 74 (17), 87 691a, 76 (7), 33 987z, 77 (5), 28 760m und 93, 106 696m, hergestellt werden, indem man z.B. Tetracarbonsäuredianhydride der Formeln

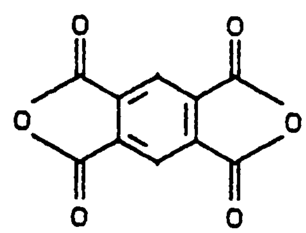
,
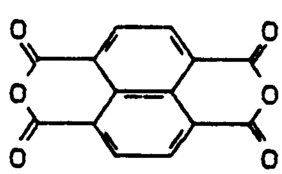
,

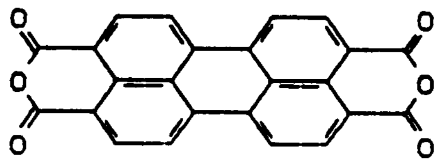
oder
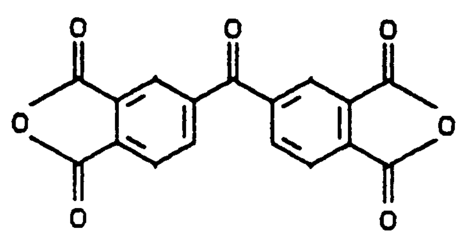

oder ein Isoindolenin der Formel

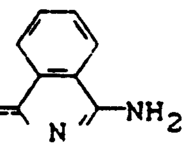

nacheinander oder gleichzeitig mit je einem Äquivalent eines Amins der Formeln

$B^1$-$Y^1$-$NH_2$ und $B^2$-$Y^2$-$NH_2$,

worin
$Y^1$ und $Y^2$ die oben angegebene Bedeutung haben,
$B^1$ und $B^2$ für

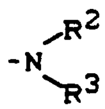 , 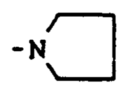 , 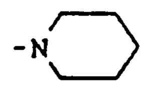 , 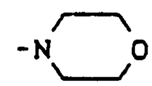

oder

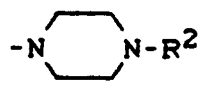

stehen und
$R^2$ und $R^3$ die oben angegebene Bedeutung haben,
kondensiert, das erhaltene Kondensationsprodukt anschließend nacheinander oder zusammen mit 2 Äquivalenten $R^1$-X, worin X für einen als Anion abspaltbaren Rest steht, quaterniert und gegebenenfalls anschließend nach bekannten Methoden, z.B. analog DE-A 37 38 948, das Anion austauscht.

Die Verbindungen der Formel (I) sind zumeist annähernd farblos oder nur schwach gefärbt. Verbindungen der Formel (I), in denen
A für

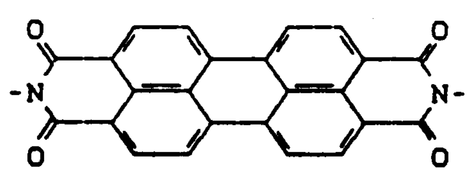

steht,
sind kationische Rotfarbstoffe.

Ladungsverstärkende Additive für elektrophotographische Toner, auch Ladungskontrollsubstanzen genannt, sind bereits bekannt. Sie werden beispielsweise in DE-A 36 04 827 und 37 38 948, EP-A 233 544, US-A 3 893 935, 3 944 493, 4 007 293, 4 079 014, 4 265 990, 4 298 672, 4 338 390, 4 394 430, 4 493 883 und JP-A 61-156 144 beschrieben.

Latente elektrostatische Bildaufzeichnungen werden dadurch entwickelt, daß der Toner auf dem elektrostatischen Bild induktiv abgeschieden wird. Die Ladungskontrollsubstanzen verstärken die kationische Ladung des Toners. Dadurch wird das Bild kräftiger und konturenschärfer.

Die in den Tonern enthaltenen Harze sind bekannt. Sie sind thermoplastisch und haben einen Erweichungspunkt zwischen 50 und 130 °C, vorzugsweise zwischen 65 und 115 °C. Beispiele für derartige Harze umfassen Polystyrol, Copolymere von Styrol mit einem Acrylat oder Methacrylat, Copolymere von

Styrol mit Butadien und/oder Acrylnitril, Polyacrylate und Polymethacrylate, Copolymere eines Acrylates oder Methacrylates mit Vinylchlorid oder Vinylacetat, Polyvinylchlorid, Copolymere von Vinylchlorid mit Vinylidenchlorid, Copolymere von Vinylchlorid mit Vinylacetat, Polyesterharze (US-A-3 590 000) Epoxyharze, Polyamide und Polyurethane.

Zusätzlich zu den Verbindungen (I) und den thermoplastischen Harzen enthalten die erfindungsgemäßen Toner in bekannten Mengen Färbungsmaterialien und gegebenenfalls magnetisch anziehbares Material. Das Färbungsmaterial kann aus einem organischen Farbstoff wie Nigrosin, Anilinblau, 2,9-Dimethylchinacridon, C.I. Disperse Red 15 (= C.I. 60 710), C.I. Solvent Red 19 (= C.I. 26 050), C.I. Pigment Blue 15 (= C.I. 74 160), C.I. Pigment Blue 22 (= C.I. 69 810) und C.I. Solvent Yellow 16 (= C.I. 12 700), oder einem anorganischen Pigment, wie Ruß, Rotblei, gelben Bleidioxid oder Chromgelb bestehen. Allgemein überschreitet die Menge des in dem Toner vorhandenen Färbungsmaterials etwa 15 Gew.-% nicht.

Das magnetisch anziehbare Material kann beispielsweise aus Eisen, Nickel, Chromoxid, Eisenoxid oder einem Ferrit der allgemeinen Formel $MFe_2O_4$, worin M ein zweiwertiges Metall, wie Eisen, Kobalt, Zink, Nickel oder Mangan darstellt, bestehen.

Die Herstellung der die Verbindungen (I) enthaltenden Toner erfolgt nach üblichen Verfahren, z.B. durch Vermischen der Bestandteile in einem Kneter und anschließendes Pulverisieren oder durch Schmelzen des thermoplastischen Harzes oder eines Gemisches der thermoplastischen Harze, anschließende feine Zerteilung einer oder mehrerer Ladungskontrollsubstanzen der Formel (I), sowie der anderen Zusätze, falls verwendet, in dem geschmolzenen Harz unter Anwendung der für diesen Zweck bekannten Misch- und Knetmaschinen, anschließend Abkühlung der Schmelze zu einer festen Masse und schließlich Vermahlen der festen Masse zu Teilchen der gewünschten Teilchengröße. Es ist auch möglich, das thermoplastische Harz und die Verbindung (I) in einem gemeinsamen Lösungsmittel zu suspendieren und die anderen Zusätze in die Suspension einzuverleiben. Die Suspension kann so als Flüssigtoner verwendet werden.

Man kann die Flüssigkeit aber auch in an sich bekannter Weise sprühtrocknen oder die Lösungsmittel abdampfen und den festen Rückstand zu Teilchen der gewünschten Teilchengröße vermahlen.

Entsprechend einer Abänderung dieses Herstellungsverfahrens wird die Ladungskontrollsubstanz der Formel (I) nicht gelöst, sondern fein in der Lösung des thermoplastischen Harzes dispergiert.

Die so erhaltene Tonerzubereitung wird dann, beispielsweise analog US-A 4 265 990, in einem xerographischen Bildaufzeichnungssystem eingesetzt.

Die verwendeten Ladungskontrollsubstanzen müssen vielseitige Anforderungen erfüllen.

1. Fähigkeit zur Entwicklung des latenten elektrosttischen Bildes zu einem farbstarken sichtbaren Bild.
2. Leichte Verteilbarkeit in der Tonerzubereitung und gleichmäßige Verteilung auf der Bildoberfläche, um ein störungsfreies, konturenscharfes, gleichförmiges Bild zu erzeugen.
3. Unempfindlichkeit gegen Feuchtigkeit.
4. Hohe Thermostabilität.
5. Beständigkeit gegenübe den heißen Mischung aus Bleidioxid und einem Vinylidenfluorid-Hexafluoropropylen-Copolymerisat-Harz (z.B. VITON$^R$E-430 von Dupont), mit der das Bild mit Hilfe einer heißen Walze fixiert werden kann. Die Beschichtungsmasse darf sich nicht durch Zersetzungsprodukte schwarz färben.

Die aus den obengenannten Patent- und Offenlegungsschriften bekannten Ladungskontrollsubstanzen erfüllen nicht alle diese Anforderungen.

Überraschenderweise zeigte sich nun, daß die Substanzen der Formel (I) gegenüber den genannten, bisher bekannten kationischen Verbindungen eine weitere Steigerung der Farbtiefe des entwickelten Bildes, eines weitere Verbesserung der Bildschärfe, eine noch geringere Empfindlichkeit gegenüber hoher Luftfeuchtigkeit und eine noch höhere Lebensdauer des Toners (mehr als 70 000 Kopien) aufweisen.

Beispiel 1

218 g Benzol-1,2,4,5-tetracarbonsäure-dianhydrid (1 Mol) werden bei 120 °C in 500 ml N-Methylpyrrolidon gelöst, mit 1 l o-Xylol, 6 g Triethylamin und unter Rückfluß am Wasserabscheider tropfenweise mit 225 g N,N-Dimethyl-1,3-diamino-propan (2,2 Mol) versetzt. Es scheidet sich ein farbloser kristalliner Niederschlag ab. Man erhitzt die Mischung noch 10 h unter Rückfluß am Wasserabscheider, setzt weitere 6 g Triethylamin zu und erhitzt weitere 6 h unter Rückfluß am Wasserabscheider. Nach dieser Zeit haben sich insgesamt 36 ml wasser abgeschieden. Die Reaktionsmischung wird unter Rühren abgekühlt. Der farblose, kristalline Niederschlag wird abgesaugt, mit Methanol gewaschen und bei 50 °C im Vakuum getrocknet. Man erhält zunächst 134 g und nach dem Eindampfen der Mutterlauge und Verrühren des Rückstandes mit 500 ml Isopropanol weitere 151 g, insgesamt also 285 g (74 % der Theorie) von der Verbindung der Formel

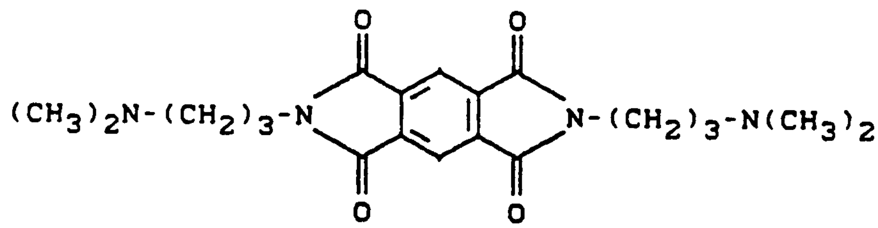

in Form farbloser Kristalle vom Schmelzpunkt 280 bis 282°C.
$C_{20}H_{26}N_4O_4$ (386,45), m/e = 386 ($M^+$).

Beispiel 2

213 g der Verbindung von Beispiel 1 (0,55 Mol) werden bei 80°C in 1,8 l Isopropanol gelöst und mit 397 g 1-Bromhexadecan (1,3 Mol) unter $N_2$ 24 h unter Rückfluß zum Sieden erhitzt, wobei sich das Umsetzungsprodukt bereits in der Siedehitze kristallin abscheidet. Nach dem Abkühlen auf Raumtemperatur wird der kristalline Niederschlag abgesaugt, mit Isopropanol gewaschen und bei 50°C im Vakuum getrocknet. Man erhält 526,5 g (96 % der Theorie) Verbindung der Formel

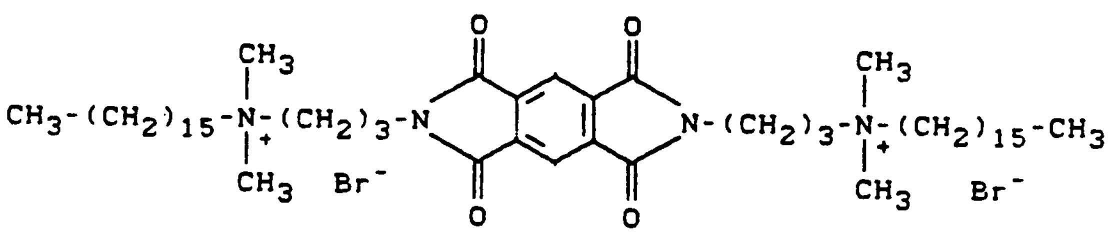

$C_{52}H_{92}Br_2N_4O_4$ (997,14)
Die Substanz ist dünnschichtchromatographisch praktisch rein und zeigt auf der fluoreszierenden Kieselgelplatte Fluoreszenzlöschung ($R_f$-Wert: 0,32, Laufmittel: 45 Vol-% Essigsäurebutylester, 33 Vol.-% Eisessig, 9 Vol-% Ameisensäure und 13 Vol-% Wasser). Sie wird in einer Strahlmühle gemahlen bis eine mittlere Korngröße von <10 µ erreicht ist.

Anwendung

2 Gew.-% Verbindung des Beispiels 2, 6 Gew.-% Ruß und 92 Gew.-% eines Styrol-Butadien-Harzes, welches 89 Gew.-% Styrol und 11 Gew.-% Butadien enthält werden im Extruder bei 100°C verschmolzen und verknetet, dann zerkleinert und gemahlen bis der partikeldurchmesser kleiner als 5 µ ist.

Diese Toner-Zubereitung wird in ein xerographisches Bildaufzeichnungssystem, wie es in US-PS 4 265 990 beschrieben ist, eingearbeitet. Dazu geht man in der Weise vor, daß man ein MYLAR®-Substrat mit einer bei Belichtung ladungserzeugenden Schicht aus Polyvinylcarbazol versieht, in das trigonales Selen frei dispergiert ist, darüber eine transparente ladungstransportierende Schicht aufbringt, die als ladungstransportierende Moleküle N,N′-Diphenyl-N,N′-bis-(3-methylphenyl)-1,1′-diphenylene-4,4′-diamin, dispergiert in einer MAKROLON®-Polycarbonatmasse, enthält.

Man erhält gestochen scharfe Bildaufzeichnungen, die denen nach US-A 4 493 883, Beispiel 1, hergestellten in der Konturenschärfe noch überlegen sind.

Analog Beispiel 1 und 2 werden die folgenden Verbindungen hergestellt:

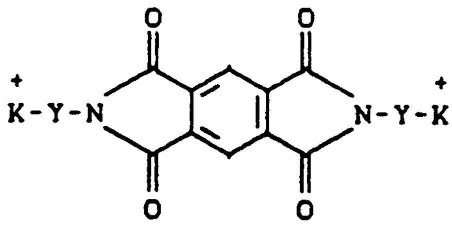 $2An^-$

| Beispiel | $\overset{+}{K}$ | $An^-$ | Y |
|---|---|---|---|
| 3 | 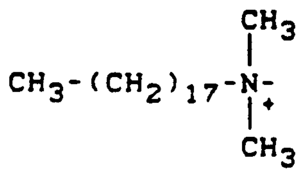 | $Br^-$ | $-(CH_2)_3-$ |
| 4 | 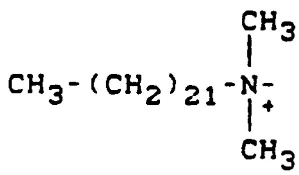 | $Br^-$ | $-CH_2-CH_2-$ |
| 5 | 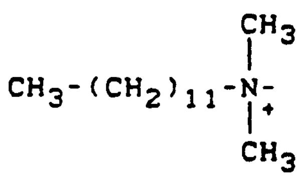 | 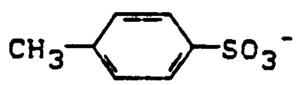 | $-(CH_2)_3-$ |
| 6 | 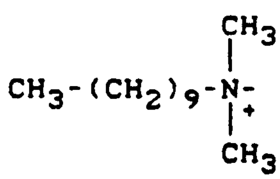 | $Cl^-$ | $-(CH_2)_3-$ |
| 7 | 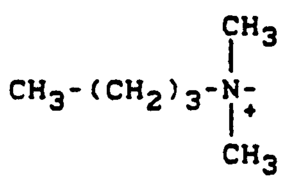 | $J^-$ | $-(CH_2)_3-$ |

| Beispiel | $\overset{+}{K}$ | $An^-$ | Y |
|---|---|---|---|
| 8 | 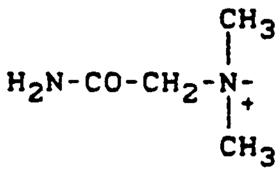 | $Cl^-$ | $-(CH_2)_3-$ |
| 9 | 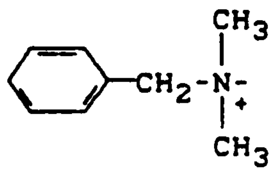 | $Cl^-$ | $-(CH_2)_3-$ |
| 10 | $(CH_3)_3\overset{+}{N}-$ | $CH_3SO_4^-$ | $-(CH_2)_3-$ |
| 11 | $(C_2H_5)_3\overset{+}{N}-$ | $C_2H_5SO_4^-$ | $-(CH_2)_2-$ |
| 12 | 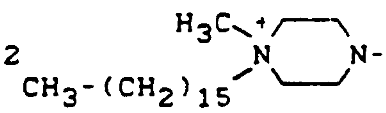 | $Br^-$ | $-CH_2-CH_2-$ |
| 13 | 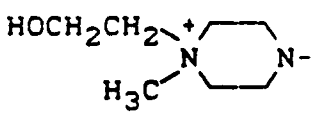 | $CH_3-COO^-$ | 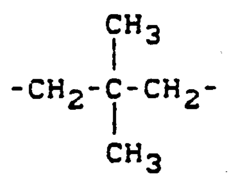 |
| 14 | 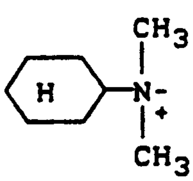 | $Br^-$ | $-(CH_2)_3-$ |

| Beispiel | $\overset{+}{K}$ | $An^-$ | Y |
|---|---|---|---|
| 15 | 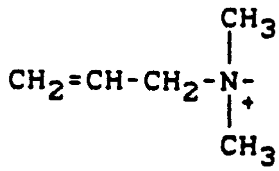 | $Cl^-$ | 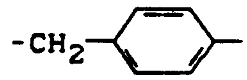 |
| 16 | 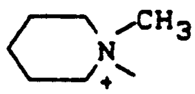 | $CH_3SO_4^-$ | 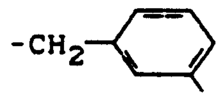 |
| 17 | 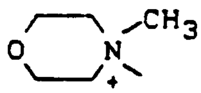 | $Cl^-$ | $-(CH_2)_3-$ |
| 18 | 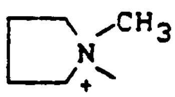 | $J^-$ | 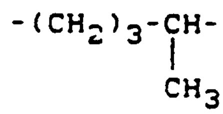 |
| 19 | 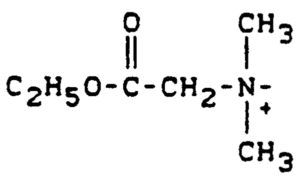 | $Br^-$ | $-(CH_2)_3-$ |

Analog Beispiel 1 und 2 werden auch die folgenden Verbindungen hergestellt:

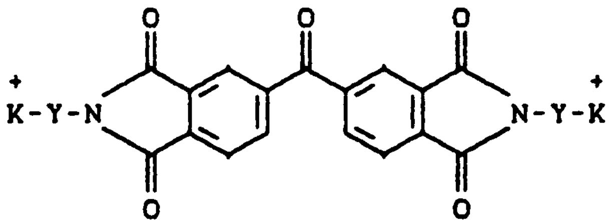 $2\ An^-$

| Beispiel | $\overset{+}{K}$- | $An^-$ | Y |
|---|---|---|---|
| 20 | 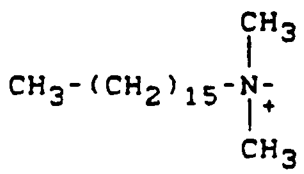 | $Br^-$ | $-(CH_2)_3-$ |
| 21 | 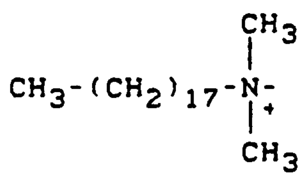 | $Br^-$ | $-(CH_2)_3$ |
| 22 | 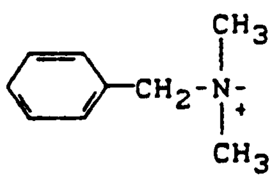 | $Cl^-$ | 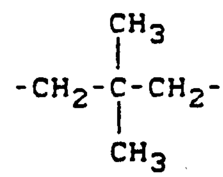 |
| 23 | $(CH_3)_3\overset{+}{N}-$ | $CH_3SO_4^-$ | 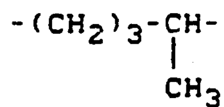 |
| 24 | 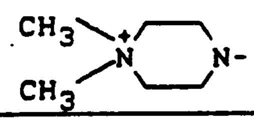 | " | $-(CH_2)_2$ |
| 25 | 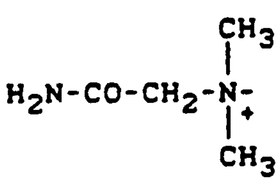 | $Cl^-$ | 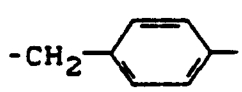 |

| Beispiel | $\overset{+}{K}$- | $An^-$ | Y |
|---|---|---|---|
| 26 | 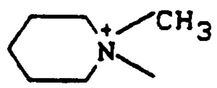 | 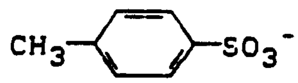 | $-(CH_2)_3-$ |
| 27 | 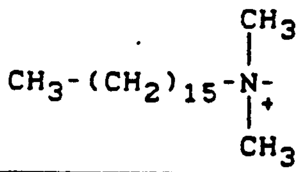 | Phosphorwolfram-molybdat | $-(CH_2)_3)-$ |
| 28 | 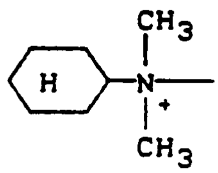 | $Br^-$ | " |

Beispiel 29

107,2 g Naphthalin-1,4,5,8-tetracarbonsäuredianhydrid (0,4 Mol) werden in 2 l Toluol suspendiert, mit 3 g Eisessig und tropfenweise unter Rühren mit 82,4 g N,N-Dimethyl-1,3-diamino-propan (0,84 Mol) versetzt und 2 h unter Rückfluß am Wasserabscheider zum Sieden erhitzt. Dann wird heiß von wenig Ungelöstem filtriert und das Filtrat abgekühlt. Der kristalline Niederschlag wird abgesaugt, mit Toluol gewaschen und bei 80 °C im Vakuum getrocknet. Man erhält 145 g (83 % der Theorie) Verbindung der Formel

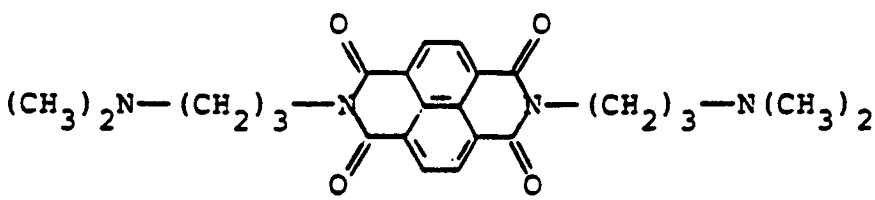

in Form metallisch glänzender goldgelber Nadeln vom Schmelzpunkt 227 °C.
$C_{24}H_{28}N_4O_4$ (436,51), m/e = 436 ($M^+$).

Beispiel 30

43,7 g Verbindung von Beispiel 29 (0,1 Mol) werden in 500 ml Isopropanol suspendiert, unter $N_2$ mit 64 g 1-Brom-hexadecan (0,21 Mol) versetzt und 20 h unter Rückfluß zum Sieden erhitzt. Der kristalline Niederschlag wird abgesaugt, mit Isopropanol gewaschen und bei 80 °C im Vakuum getrocknet. Man erhält 97 g (92,6 % der Theorie) Verbindung der Formel

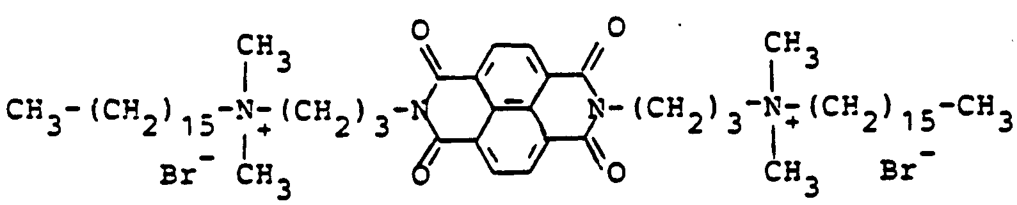

$C_{56}H_{94}Br_2N_4O_4$ (1 047,20)
Die Substanz ist dünnschichtchromatographisch fast einheitlich und zeigt auf der fluoreszierenden Kieselgelplatte Fluoreszenzlöschung. $R_f$-Wert: 0,31 (Laufmittel: 45 Vol-% Essigsäurebutylester, 33 Vol-% Eisessig, 9 Vol-% Ameisensäure und 13 Vol-% Wasser). Sie wird in einer Strahlmühle gemahlen, bis eine mittlere Korngröße von <10 µ erreicht ist.

Anwendung

100 g Styrol-n-butylmethacrylat-Copolymer (Mol: 50 000) und 2 g Verbindung von Beispiel 30 werden in einem Kneter bei 100 °C gleichmäßig vermischt. Nach dem Abkühlen pulverisiert man das Harz in einer Strahlmühle auf eine mittlere Kornfeinheit von 5 µ. 5 g dieses Tonerpulvers werden mit 95 g eines Carriermaterials aus Eisen mit Polymerbeschichtung durch Rotation aufgeladen und die Ladung nach der Blow-off-Methode bestimmt. Sie beträgt 25,3 µC/g und ist nach 70 000 Kopien noch unverändert hoch.
Die gestochen scharfen Bildaufzeichnungen sind den mit Ladungskontrollsubstanzen aus DE-A 36 04 827, Beispiele 1 und 34, erzeugten in der Konturenschärfe überlegen.

Analog Beispiel 29 und 30 werden die folgenden Verbindungen hergestellt:

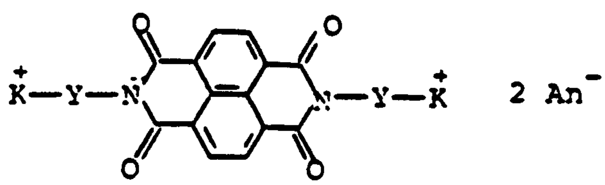

| Beispiel | $\overset{+}{K}$- | $An^-$ | Y |
|---|---|---|---|
| 31 | $CH_3-(CH_2)_{17}-\overset{+}{N}(CH_3)_2-$ | $Br^-$ | $-(CH_2)_3-$ |
| 32 | $CH_3-(CH_2)_{21}-\overset{+}{N}(CH_3)_2-$ | $Br^-$ | $-CH_2-CH_2-$ |
| 33 | $CH_3-(CH_2)_{11}-\overset{+}{N}(CH_3)_2-$ | 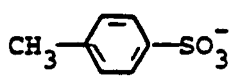 | $-(CH_2)_3-$ |
| 34 | $CH_3-(CH_2)_9-\overset{+}{N}(CH_3)_2-$ | Phosphorwolfram-molybdat | " |
| 35 | $CH_3(CH_2)_3-\overset{+}{N}(H)(CH_3)-$ | $J^-$ | " |
| 36 | $H_2N-CO-CH_2-\overset{+}{N}(CH_3)_2-$ | Silico-molybdat | $-(CH_2)_2-$ |
| 37 | 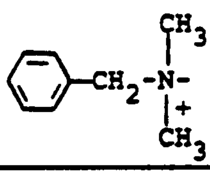 | $Cl^-$ | $-(CH_2)_3-$ |
| 38 | $(CH_3)_3\overset{+}{N}-$ | $CH_3SO_4^-$ | " |

| Beispiel | $\overset{+}{K}$- | $An^-$ | Y |
|---|---|---|---|
| 39 | $(C_2H_5)_3\overset{+}{N}$- | $C_2H_5SO_4^-$ | $-(CH_2)_2-$ |
| 40 | 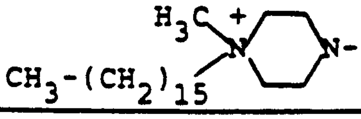 | $Br^-$ | $-CH_2-CH_2-$ |
| 41 | 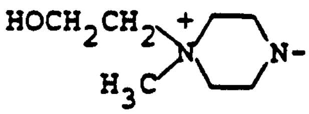 | $CH_3-COO^-$ | 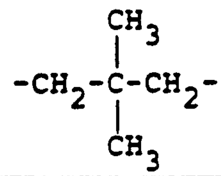 |
| 42 | 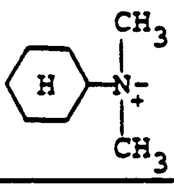 | $Br^-$ | $-(CH_2)_3-$ |
| 43 | 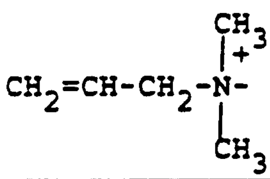 | $Cl^-$ | 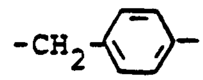 |
| 44 | 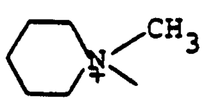 | $CH_3SO_4^-$ | 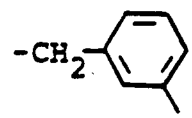 |
| 45 | 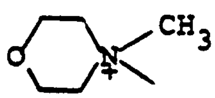 | $Cl^-$ | $-(CH_2)_3-$ |
| 46 | 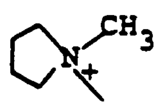 | $J^-$ | 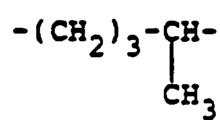 |
| 47 | 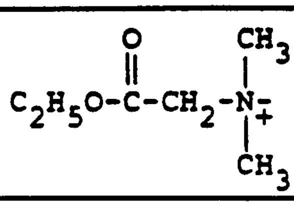 | $Br^-$ | $-(CH_2)_3-$ |

Analog Beispiel 30 wird aus dem in DE-A 35 35 496, Beispiel 330, beschriebenen Vorprodukt die Verbindung der folgenden Formel hergestellt:

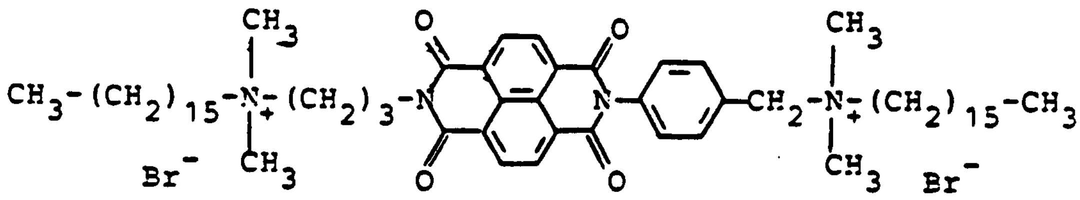

**Beispiel 48**

Beispiel 49

296 g Perylentetracarbonsäuredianhydrid (0,5 Mol) werden in 2 l Isopropanol suspendiert, mit 4 g Eisessig und bei Siedetemperatur unter Rühren tropfenweise mit 103 g N,N-Dimethyl-1,3-diamino-propan (1 Mol) versetzt. Die Mischung wird 68 h unter Rückfluß zum Sieden erhitzt. Eine Probe der Suspension löst sich vollständig in verdünnter Salzsäure. Nach dem Abkühlen auf Raumtemperatur wird der kristalline Niederschlag abgesaugt, mit Isopropanol gewaschen und bei 80 ° C im Vakuum getrocknet. Man erhält 273 g Verbindung der Formel

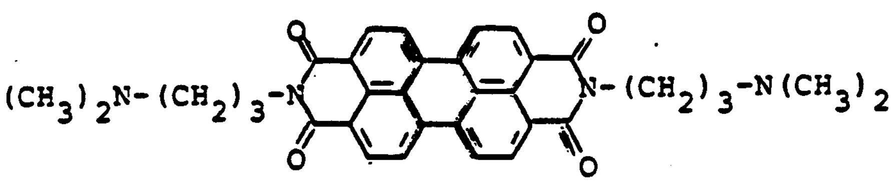

als dunkles Kristallpulver, das oberhalb 300 ° C schmilzt. Ein Massenspektrum (DCI-Anregung) zeigt die Masse 561 $(M+H)^+$.
$C_{34}H_{32}N_4O_4$ (560,65).

Beispiel 50

56 g der Verbindung von Beispiel 49 (0,1 Mol) werden in 500 ml N-Methyl-pyrrolidon auf 100 ° C erwärmt und unter Stickstoffschutz und unter Rühren mit 64,1 g 1-Bromhexadecan (0,21 Mol) versetzt. Die Mischung wird 48 h auf 120 ° C erhitzt und dann abgekühlt. Der kristalline Niederschlag wird abgesaugt, mit Isopropanol gewaschen und bei 80 ° C im Vakuum getrocknet. Man erhält 106 g (90 % der Theorie) Verbindung der Formel

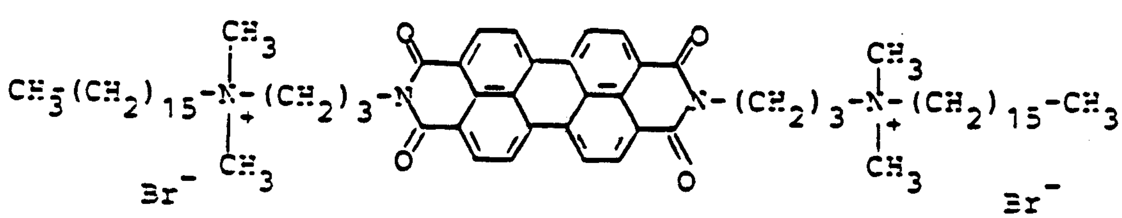

$C_{66}H_{98}Br_2N_4O_4$ (1 171,34)
Die Substanz ist dünnschichtchromatographisch praktisch rein und zeigt auf der fluoreszierenden Kieselgelplatte Fluoreszenzlöschung. $R_f$-Wert: 0,17 (Laufmittel: 45 Vol-% Essigsäurebutylester, 33 Vol-% Eisessig, 9 Vol-% Ameisensäure und 13 Vol-% Wasser).

Anwendung

2-Gew.-% Verbindung des Beispiels 50, 6 Gew.-% Ruß und 92 Gew.-% eines Styrol-Butadien-Harzes, welches 89 Gew.-% Styrol und 11 Gew.-% Butadien enthält, werden im Extruder bei 100 ° C verschmolzen und verknetet, dann zerkleinert und gemahlen, bis der Partikeldurchmesser kleiner als 5 µ ist.

Diese Tonerzubereitung wird in ein xerographisches Bildaufzeichnungssystem, wie es in US-A-4 265 990 beschrieben ist, eingearbeitet. Dazu geht man in der Weise vor, daß man ein MYLAR®-Substrat mit einer bei Belichtung ladungserzeugenden Schicht aus Polyvinylcarbazol versieht, in das trigonales Selen frei dispergiert ist, darüber eine transparente ladungstransportierende Schicht aufbringt, die als ladungstransportierende Moleküle N,N′-Diphenyl-N,N′-bis-(3-methylphenyl)-1,1′-diphenylene-4,4′-diamin, dispergiert in einer MAKROLON®-Polycarbonatmasse, enthält.

Man erhält gestochen scharfe Bildaufzeichnungen, die denen nach DE-A 36 04 817, Beispiel 1, hergestellten in der Farbsättigung überlegen sind.

Analog Beispiel 49 und 50 werden die folgenden Verbindungen hergestellt:

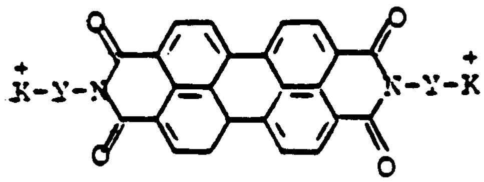

2 $An^-$

| Beispiel | $\overset{+}{K}$- | $An^-$ | Y |
|---|---|---|---|
| 51 | $CH_3$<br>\|<br>$CH_3-(CH_2)_{17}-N-$<br>\|+<br>$CH_3$ | $Br^-$ | $-(CH_2)_3-$ |
| 52 | $CH_3$<br>\|<br>$CH_3-(CH_2)_{21}-N-$<br>\|+<br>$CH_3$ | $Br^-$ | $-CH_2-CH_2-$ |
| 53 | $CH_3$<br>\|<br>$CH_3-(CH_2)_{11}-N-$<br>\|+<br>$CH_3$ | 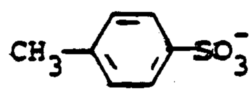 | $-(CH_2)_3-$ |
| 54 | $CH_3$<br>\|<br>$CH_3-(CH_2)_9-N-$<br>\|+<br>$CH_3$ | $Cl^-$ | " |
| 55 | $CH_3$<br>\|<br>$CH_3(CH_2)_3-N-$<br>\|+<br>$CH_3$ | $J^-$ | " |
| 56 | $CH_3$<br>\|<br>$H_2N-CO-CH_2-N-$<br>\|+<br>$CH_3$ | $Cl^-$ | $-(CH_2)_2-$ |

| Beispiel | $\overset{+}{K}$- | $An^-$ | Y |
|---|---|---|---|
| 57 | 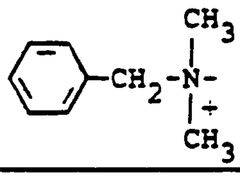 | $Cl^-$ | $-(CH_2)_3-$ |
| 58 | $(CH_3)_3\overset{+}{N}-$ | $CH_3SO_4^-$ | " |
| 59 | $(C_2H_5)_3\overset{+}{N}-$ | $C_2H_5SO_4^-$ | $-(CH_2)_2-$ |
| 60 | 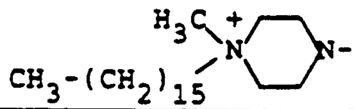 | $Br^-$ | $-CH_2-CH_2-$ |
| 61 | 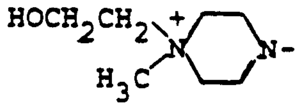 | $CH_3-COO^-$ | 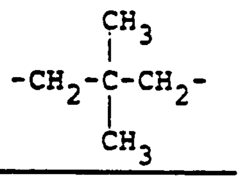 |
| 62 | 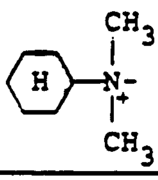 | $Br^-$ | $-(CH_2)_3-$ |
| 63 | 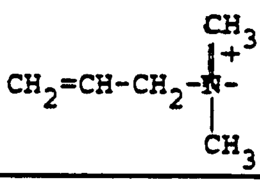 | $Cl^-$ | 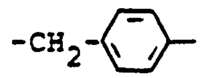 |
| 64 | 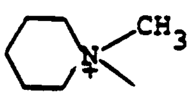 | $CH_3SO_4^-$ | 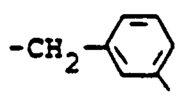 |
| 65 | 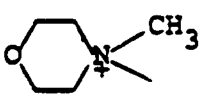 | $Cl^-$ | $-(CH_2)_3-$ |

| Beispiel | $K^+$- | $An^-$ | Y |
|---|---|---|---|
| 66 | 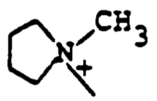 | $J^-$ | 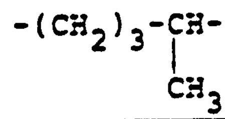 |
| 67 | 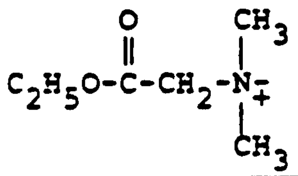 | $Br^-$ | $-(CH_2)_3-$ |

Beispiel 68

15,8 g 3-Amino-1-imino-isoindolenin (92 %ig, 0,1 Mol) werden in 100 ml Isopropanol in der Siedehitze unter Rühren tropfenweise mit 23,5 g N,N-Dimethyl-1,3-diaminopropan (0,23 Mol) versetzt und 4 h unter Rückfluß zum Sieden erhitzt, wobei $NH_3$ entweicht. Nach dieser Zeit zeigt ein Dünnschichtchromatogramm (Laufmittel wie in Beispiel 2) eine vollständige, einheitliche Umsetzung zu

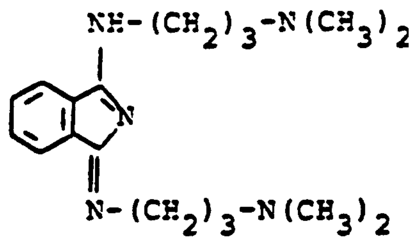

$C_{18}H_{29}N_5$ (315,46), m/e = 315 ($M^+$).

Diese Verbindung wird ohne Zwischenisolierung zu der in Beispiel 69 beschriebenen Verbindung weiterverarbeitet.

Beispiel 69

Die Lösung der in Beispiel 68 hergestellten Verbindung (ca. 0,1 Mol) wird mit 350 ml Isopropanol verdünnt, siedend heiß unter $N_2$ und unter Rühren tropfenweise mit 70 g 1-Brom-hexadecan (0,23 Mol) versetzt und 10 h unter Rückfluß zum Sieden erhitzt. Nach mehrtägigem Stehen bei Raumtemperatur wird der farblose, kristalline Niederschlag abgesaugt, mit Aceton ausgerührt, abgesaugt, mit Aceton gewaschen und bei 50 ° C im Vakuum getrocknet. Man erhält 67 g (72,3 % der Theorie) Verbindung der Formel

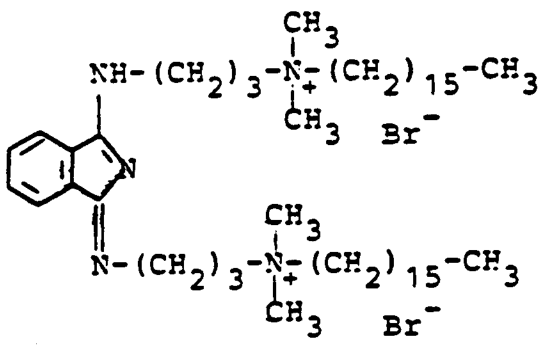

$C_{50}H_{95}Br_2N_5$ (926,15)

Die Substanz ist dünnschichtchromatographisch praktisch rein und zeigt auf der fluoreszierenden Kieselgelplatte Fluoreszenzlöschung.

$R_f$-Wert: 0,42 (Laufmittel wie in Beispiel 2).

Sie wird in einer Strahlmühle gemahlen, bis eine mittlere Korngröße von <10 µ erreicht ist.

Anwendung

2 Gew.-% Verbindung des Beispiels 69, 6 Gew.-% Ruß und 92 Gew.-% eines Styrol-Butadien-Harzes, welches 89 Gew.-% Styrol und 11 Gew.-% Butadien enthält, werden im Extruder bei 100 °C verschmolzen und verknetet, dann zerkleinert und gemahlen, bis der Partikeldurchmesser kleiner als 5 µ ist.

Diese Tonerzubereitung wird in ein xerographisches Bildaufzeichnungssystem, wie es in US-A-4 265 990 beschrieben ist, eingearbeitet. Dazu geht man in der Weise vor, daß man ein MYLAR®-Substrat mit einer bei Belichtung ladungserzeugenden Schicht aus Polyvinylcarbazol versieht, in das trigonales Selen frei dispergiert ist, darüber eine transparente ladungstransportierende Schicht aufbringt, darüber eine transparente ladungstransportierende Schicht aufbringt, die als ladungstransportierende Moleküle N,N'-Diphenyl-N,N'-bis-(3-methylphenyl)-1,1'-diphenylene-4,4'-diamin, dispergiert in einer MAKROLON®-Polycarbonatmasse, enthält.

Man erhält gestochen scharfe Bildaufzeichnungen.

Analog Beispiel 68 und 69 werden die folgenden Verbindungen hergestellt:

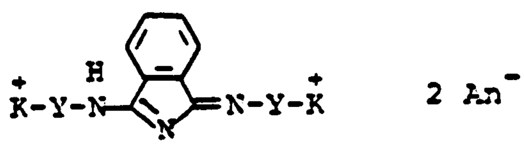

| Beispiel | $\overset{+}{K}$- | $An^-$ | Y |
|---|---|---|---|
| 70 | $CH_3$<br>\|<br>$CH_3-(CH_2)_{17}-N-$<br>\|+<br>$CH_3$ | $Br^-$ | $-(CH_2)_3-$ |
| 71 | $CH_3$<br>\|<br>$CH_3-(CH_2)_{21}-N-$<br>\|+<br>$CH_3$ | $Br^-$ | $-CH_2-CH_2-$ |
| 72 | $CH_3$<br>\|<br>$CH_3-(CH_2)_{11}-N-$<br>\|+<br>$CH_3$ | 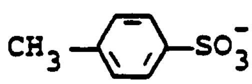 | $-(CH_2)_3-$ |
| 73 | $CH_3$<br>\|<br>$CH_3-(CH_2)_9-N-$<br>\|+<br>$CH_3$ | Phosphorwolfram-molybdat | " |
| 74 | $CH_3$<br>\|<br>$CH_3(CH_2)_3-N-$<br>\|+<br>$CH_3$ | $J^-$ | " |
| 75 | $CH_3$<br>\|<br>$H_2N-CO-CH_2-N-$<br>\|+<br>$CH_3$ | $Cl^-$ | $-(CH_2)_2-$ |

| Beispiel | $\overset{+}{K}$- | $An^-$ | Y |
|---|---|---|---|
| 76 | 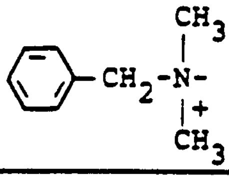 | $Cl^-$ | $-(CH_2)_3-$ |
| 77 | $(CH_3)_3\overset{+}{N}-$ | $CH_3SO_4^-$ | " |
| 78 | $(C_2H_5)_3\overset{+}{N}-$ | $C_2H_5SO_4^-$ | $-(CH_2)_2-$ |
| 79 | 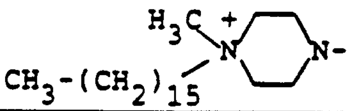 | $Br^-$ | $-CH_2-CH_2-$ |
| 80 | 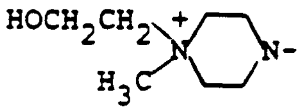 | $CH_3-COO^-$ | 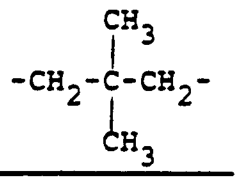 |
| 81 | 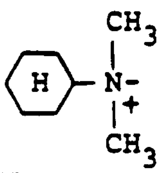 | $Br^-$ | $-(CH_2)_3-$ |
| 82 | 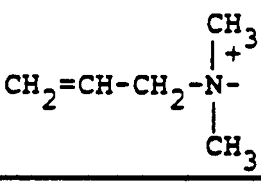 | $Cl^-$ | 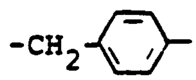 |
| 83 | 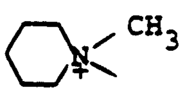 | $CH_3SO_4^-$ | 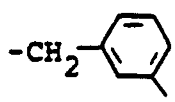 |

| Beispiel | $\overset{+}{K}$- | $An^-$ | Y |
|---|---|---|---|
| 84 | 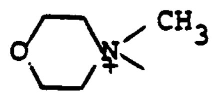 | $Cl^-$ | $-(CH_2)_3-$ |
| 85 | 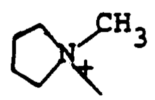 | $J^-$ | 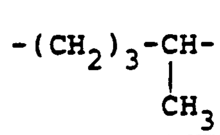 |
| 86 | 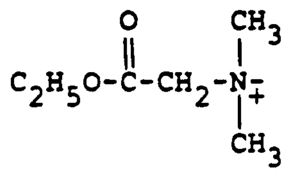 | $Br^-$ | $-(CH_2)_3-$ |

## Patentansprüche

1. Elektrophotographische Toner, die ein die kationische Ladung verstärkendes Additiv der allgemeinen Formel

$$(X^1)^- \; \overset{+}{K}{}^1\text{-}Y^1\text{-}A\text{-}Y^2\text{-}\overset{+}{K}{}^2 \; (X^2)^-$$

enthalten, worin

| | |
|---|---|
| $(X^1)^-$ und $(X^2)^-$ | unabhängig voneinander für ein Anion, |
| $\overset{+}{K}{}^1$ und $\overset{+}{K}{}^2$ | unabhängig voneinander für |

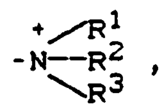 ,

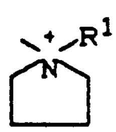 , 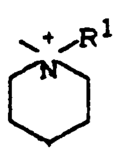 , 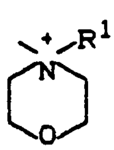 ,

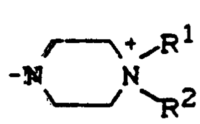 oder 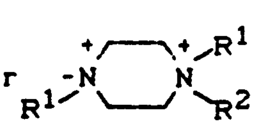 $(X^1)^-$ ,

| | |
|---|---|
| $R^1$ | für $C_1$-$C_{22}$-Alkyl, Benzyl, Phenyl, Cyclohexyl oder Allyl, |
| $R^2$ | für Wasserstoff oder $C_1$-$C_4$-Alkyl, |
| $R^3$ | für $C_1$-$C_4$-Alkyl und |
| $Y^1$ und $Y^2$ | unabhängig voneinander für $C_2$-$C_5$-Alkylen oder $_*C_6H_4$-$CH_2$- (-m oder -p) stehen, wobei die mit * gekennzeichnete Bindung mit A verbunden ist, und |
| A | ein Tetracarbonsäurediimid der Formeln |

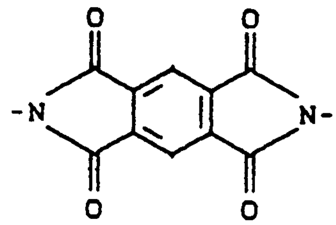 , 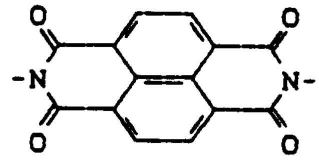 ,

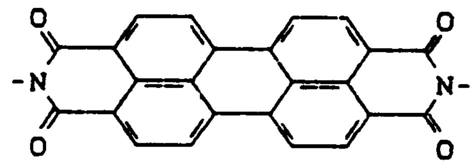 oder

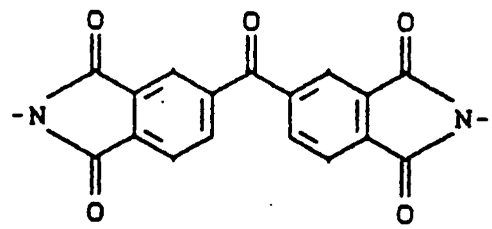

oder ein 3-Amino-1-imino-isoindolenin der Formel

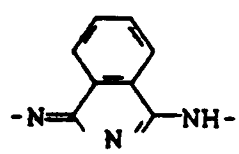

bedeutet.

**2.** Elektrophotographische Toner nach Anspruch 1, dadurch gekennzeichnet, daß

$R^1$ für $C_1$-$C_{22}$-Alkyl, Carbamoyl-$C_1$-$C_2$-alkyl, $C_1$-$C_4$-Alkoxycarbonyl-$C_1$-$C_2$-alkyl, Benzyl, Cyclohexyl oder Allyl,

$R^2$ und $R^3$ unabhängig voneinander für $C_1$-$C_4$-Alkyl und

$(X^1)^-$, $(X^2)^-$, $Y^1$, $Y^2$ und A die gleiche Bedeutung wie in Anspruch 1 haben.

**3.** Elektrophotographische Toner nach Anspruch 1, dadurch gekennzeichnet, daß

A für ein aromatisches Tetracarbonsäurediimid der Formel

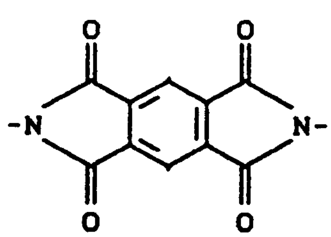

steht und die übrigen Symbole die in Anspruch 1 angegebene Bedeutung haben.

4. Elektrophotographische Toner nach Anspruch 1, dadurch gekennzeichnet, daß
A für ein aromatisches Tetracarbonsäurediimid der Formel

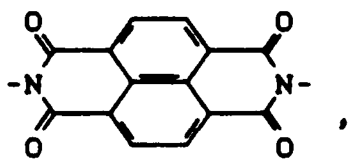

steht und die übrigen Symbole die in Anspruch 1 angegebene Bedeutung haben.

5. Elektrophotographische Toner nach Anspruch 1, dadurch gekennzeichnet, daß
A für ein aromatisches Tetracarbonsäurediimid der Formel

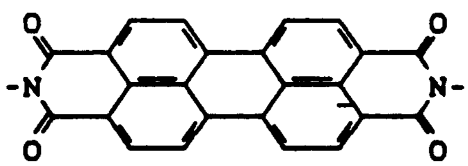

steht und die übrigen Symbole die in Anspruch 1 angegebene Bedeutung haben.

6. Elektrophotographische Toner nach Anspruch 1, dadurch gekennzeichnet, daß
A für ein aromatisches Tetracarbonsäurediimid der Formel

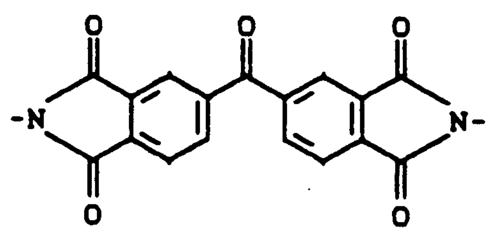

steht und die übrigen Symbole die in Anspruch 1 angegebene Bedeutung haben.

7. Elektrophotographische Toner nach Anspruch 1, dadurch gekennzeichnet, daß
A für ein aromatisches 3-Amino-1-imino-isoindolenin der Formel

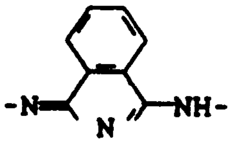

steht und die übrigen Symbole die in Anspruch 1 angegebene Bedeutung haben.

8. Elektrophotographische toner nach Anspruch 1, dadurch gekennzeichnet, daß sie außer dem die kationische Ladung verstärkenden Additiv Harz- und Pigmentpartikel enthalten.

9. Verwendung von Verbindungen der Formel des Anspruchs 1 als die positive Ladung verstärkende Additive in elektrophotographischen Tonern.

**10.** Verbindungen der allgemeinen Formel

$(X^1)^- \overset{+}{K}{}^3\text{-}Y^3\text{-}A\text{-}Y^4\text{-}\overset{+}{K}{}^4\ (X^2)^-$

worin

| | |
|---|---|
| $\overset{+}{K}{}^3$ und $\overset{+}{K}{}^4$ | unabhängig voneinander für |

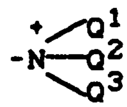

oder

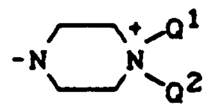

,

| | |
|---|---|
| $Q^1$ | für $C_8$-$C_{18}$-Alkyl, |
| $Q^2$ | für Wasserstoff, Methyl oder Ethyl, |
| $Y^3$ und $Y^4$ | unabhängig voneinander für $C_2$-$C_5$-Alkylen stehen, und |
| $(X^1)^-$, $(X^2)^-$ und A | die gleiche Bedeutung wie in Anspruch 1 besitzen. |

## Claims

**1.** Electrophotographic toners containing an additive which increases the cationic charge and has the general formula

$(X^1)^- \overset{+}{K}{}^1\text{-}Y^1\text{-}A\text{-}Y^2\text{-}\overset{+}{K}{}^2\ (X^2)^-$

wherein

| | |
|---|---|
| $(X^1)^-$ and $(X^2)^-$ | independently of one another represent an anion, |
| $\overset{+}{K}{}^1$ and $\overset{+}{K}{}^2$ | independently of one another represent |

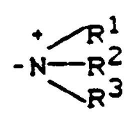

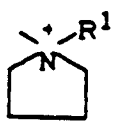

,

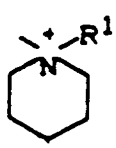

,

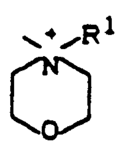

,

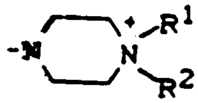

or

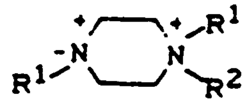

$(X^1)^-$ ,

| | |
|---|---|
| $R^1$ | represents $C_1$-$C_{22}$-alkyl, benzyl, phenyl, cyclohexyl or allyl, |
| $R^2$ | represents hydrogen or $C_1$-$C_4$-alkyl, |
| $R^3$ | represents $C_1$-$C_4$-alkyl and |
| $Y^1$ and $Y^2$ | independently of one another represent $C_2$-$C_5$-alkylene or $_*C_6H_4$-$CH_2$- (-m or -p), the bond marked with * being attached to A, and |

A denotes a tetracarborylic acid diimide of the formulae

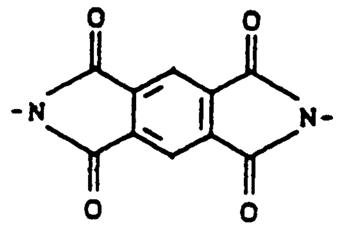
,
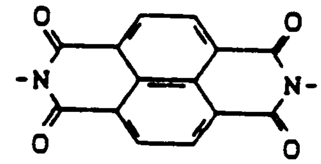
,

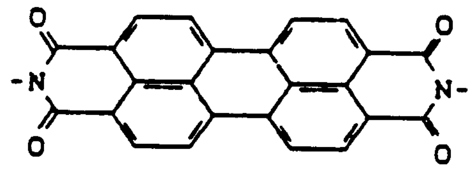
or

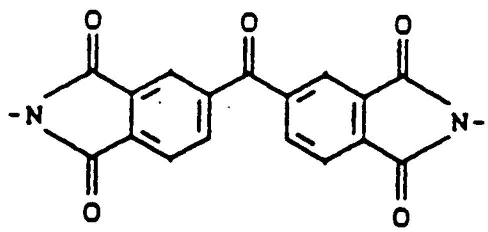

or a 3-amino-1-imino-isoindolenine of the formula

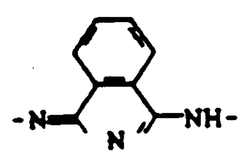

**2.** Electrophotographic toners according to Claim 1, characterized in that

| | |
|---|---|
| $R^1$ | represents $C_1$-$C_{22}$-alkyl, carbamoyl-$C_1$-$C_2$-alkyl, $C_1$-$C_4$-alkoxycarbonyl-$C_1$-$C_2$-alkyl, benzyl, cyclohexyl or allyl, |
| $R^2$ and $R^3$ | independently of one another represent $C_1$-$C_4$-alkyl and |
| $(X^1)^-$, $(X^2)^-$, $Y^1$, $Y^2$ and A | have the same meaning as in Claim 1. |

**3.** Electrophotographic toners according to Claim 1, characterized in that
A represents an aromatic tetracarboxylic acid diimide of the formula

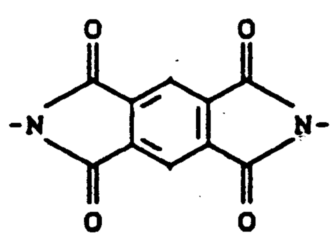

and the remaining symbols have the meaning indicated in Claim 1.

4. Electrophotographic toners according to Claim 1, characterized in that
A represents an aromatic tetracarboxylic acid diimide of the formula

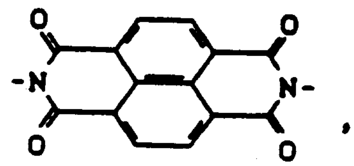

and the remaining symbols have the meaning indicated in Claim 1.

5. Electrophotographic toners according to Claim 1, characterized in that
A represents an aromatic tetracarboxylic acid diimide of the formula

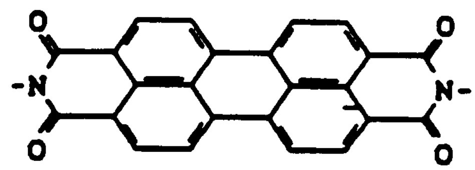

and the remaining symbols have the meaning indicated in Claim 1.

6. Electrophotographic toners according to Claim 1, characterized in that
A represents an aromatic tetracarboxylic acid diimide of the formula

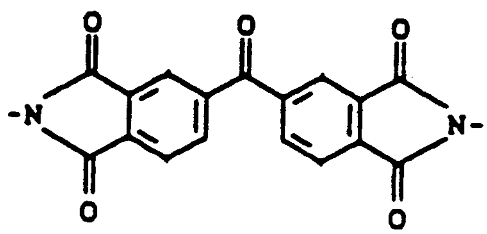

and the remaining symbols have the meaning indicated in Claim 1.

7. Electrophotographic toners according to Claim 1, characterized in that
A represents an aromatic 3-amino-1-imino-isoindolenine of the formula

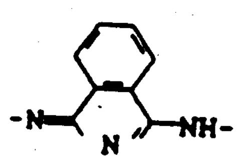

and the remaining symbols have the meaning indicated in Claim 1.

8. Electrophotographic toners according to Claim 1, characterized in that, in addition to the additive which increases the cationic charge, they contain resin and pigment particles.

**9.** The use, of compounds of the, formula of Claim 1 as additives which increase the positive charge in electrophotographic toners.

**10.** Compounds of the general formula

$(X^1)^- \overset{+}{K}{}^3\text{-}Y^3\text{-}A\text{-}Y^4\text{-}\overset{+}{K}{}^4\ (X^2)^-$

wherein

$\overset{+}{K}{}^3$ and $\overset{+}{K}{}^4$ independently of one another represent

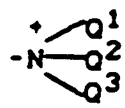

or

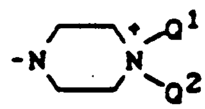

| | |
|---|---|
| $Q^1$ | represents $C_8$-$C_{18}$-alkyl, |
| $Q^2$ | represents hydrogen, methyl or ethyl, |
| $Y^3$ and $Y^4$ | independently of one another represent $C_2$-$C_5$-alkylene and |
| $(X^1)^-$, $(X^2)^-$ and A | have the same meaning as in Claim 1. |

## Revendications

**1.** Toners électrophotographiques, qui contiennent un additif renforçant la charge cationique, de formule générale

$(X^1)^- \overset{+}{K}{}^1\text{-}Y^1\text{-}A\text{-}Y^2\text{-}\overset{+}{K}{}^2\ (X^2)^-$

dans laquelle

$(X^1)^-$ et $(X^2)^-$ représentent indépendamment l'un de l'autre un anion,

$\overset{+}{K}{}^1$ et $\overset{+}{K}{}^2$ représentent indépendamment l'un de l'autre

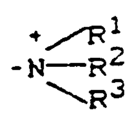,

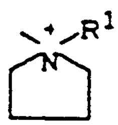, 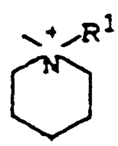, 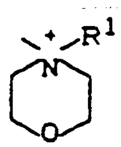,

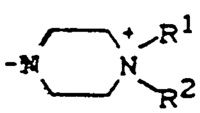 ou 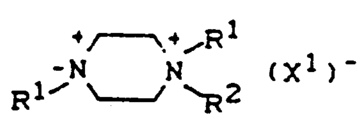,

$R^1$ représente un groupe alkyle en $C_1$-$C_{22}$, benzyle, phényle, cyclohexyle ou allyle,

$R^2$ représente un atome d'hydrogène ou un groupe alkyle en $C_1$-$C_4$,

$R^3$ représente un groupe alkyle en $C_1$-$C_4$ et

$Y^1$ et $Y^2$ représentent indépendamment l'un de l'autre un groupe alkylène en $C_2$-$C_5$ ou $*C_6H_4$-$CH_2$- (m ou p), la liaison repérée par * étant liée à A

A représente un diimide d'acide tétracarboxylique de formule

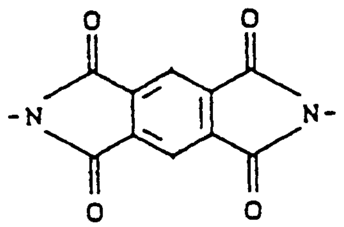
,
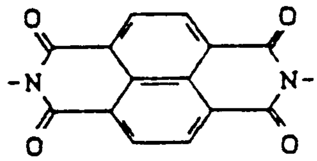
,

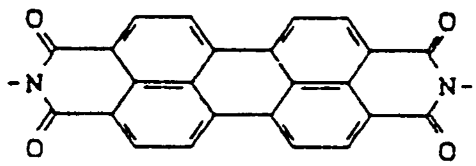
ou

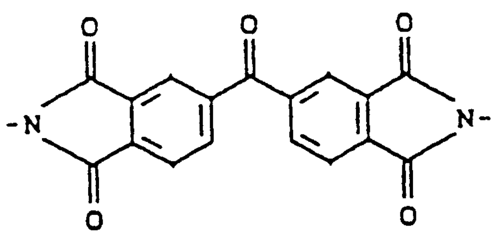

ou une 3-amino-1-imino-isoindolénine de formule

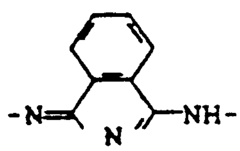

2. Toners électrophotographiques selon la revendication 1, caractérisés en ce que

$R^1$ représente un groupe alkyle en $C_1$-$C_{22}$, carbamoyl-alkyle en $C_1$ ou $C_2$, (alcoxy en $C_1$-$C_4$) carbonyl-alkyle en $C_1$ ou $C_2$, benzyle, cyclohexyle ou allyle,

$R^2$ et $R^3$ représentent indépendamment l'un de l'autre un groupe alkyle en $C_1$-$C_4$ et

$(X^1)^-$, $(X^2)^-$, $Y^1$, $Y^2$ et A ont la même signification qu'à la revendication 1.

3. Toners électrophotographiques selon la revendication 1, caractérisés en ce que

A représente un diimide d'acide tétracarboxylique aromatique de formule

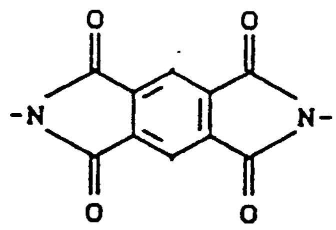

et les autres symboles ont la signification indiquée dans la revendication 1.

4. Toners électrophotographiques selon la revendication 1, caractérisés en ce que A représente un diimide d'acide tétracarboxylique aromatique de formule :

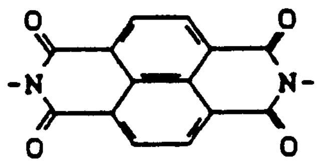

,

et les autres symboles ont la signification indiquée dans la revendication 1.

5. Toners électrophotographiques selon la revendication 1, caractérisés en ce que A représente un diimide d'acide tétracarboxylique aromatique de formule

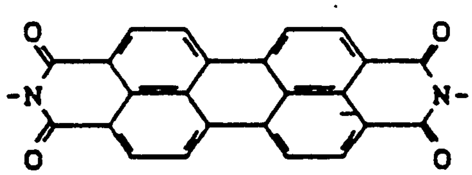

et les autres symboles ont la signification indiquée à la revendication 1.

6. Toners électrophotographiques selon la revendication 1, caractérisés en ce que A représente un diimide d'acide tétracarboxylique aromatique de formule

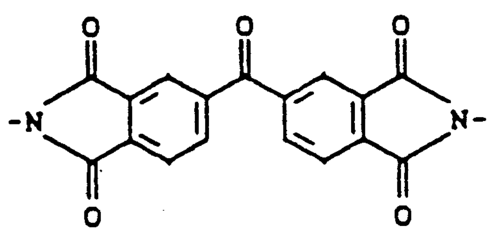

et les autres symboles ont la signification indiquée à la revendication 1.

7. Toners électrophotographiques selon la revendication 1, caractérisés en ce que A représente une 3-amino-1-imino-isoindolénine aromatique de formule

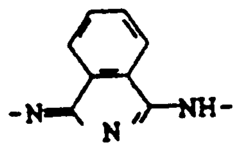

et les autres symboles ont la signification indiquée dans la revendication 1.

**8.** Toners électrophotographiques selon la revendication 1, caractérisés en ce qu'ils contiennent des particules de résine et de pigment en plus de l'additif renforçant la charge cationique.

**9.** Utilisation de composés de formule de la revendication 1, comme additifs renforçant la charge positive dans des toners électrophotographiques.

**10.** Composés de formule générale

$(X^1)^- \overset{+}{K}{}^3\text{-}Y^3\text{-}A\text{-}Y^4\text{-}\overset{+}{K}{}^4 \ (X^2)^-$

dans laquelle
$\overset{+}{K}{}^3$ et $\overset{+}{K}{}^4$ représentent indépendamment l'un de l'autre

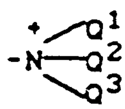

ou

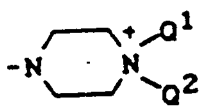

$Q^1$ représente un groupe alkyle en $C_8$-$C_{18}$,
$Q^2$ représente un atome d'hydrogène ou un groupe méthyle ou éthyle,
$Y^3$ et $Y^4$ représentent indépendamment l'un de l'autre un groupe alkylène en $C_2$-$C_5$ et
$(X^1)^-$, $(X^2)^-$ et A ont la même signification que dans la revendication 1.